# EUROPEAN PATENT APPLICATION

(11) **EP 3 950 921 A1**
(43) Date of publication of application: **09.02.2022**
(21) Application number: 20778817.5
(22) Date of filing: 24.03.2020
(51) Int. Cl.: C12M 1/26, C12M 3/06

(54) **CELL SUSPENSION TREATMENT APPARTUS**

(30) Priority: 26.03.2019 JP 2019057953; 26.03.2019 JP 2019057954; 26.03.2019 JP 2019057955
(71) Applicant: KYOTO SEISAKUSHO CO., LTD., Kyoto-shi, Kyoto 6130916 (JP); Megakaryon Corporation, Kyoto-shi, Kyoto 6008813 (JP)
(72) Inventor: HIGUCHI, Akira, Kyoto-shi, Kyoto 613-0916 (JP); NAITO, Hiroyuki, Kyoto-shi, Kyoto 613-0916 (JP); OKAMOTO, Haruki, Kyoto-shi, Kyoto 600-8813 (JP)
(74) Representative: Eisenführ Speiser
(86) International application number: PCT/JP2020/013121
(87) International publication number: WO 2020/196551

(57) **Abstract**

A cell suspension treatment apparatus has a circulation circuit and a filling liquid supply source that supplies a filling liquid to the circulation circuit. The circulation circuit includes a hollow fiber membrane filter, a reservoir, a pump that is provided on a downstream side of the reservoir and on an upstream side of the hollow fiber membrane filter, and a valve that is provided on the downstream side of the reservoir and on an upstream side of the pump. The filling liquid supply source is connected to a portion of the circulation circuit between the first valve and the pump. After the concentrated cell suspension is stored in the reservoir, in a state where the valve is closed and the pump is driven, the filling liquid supply source starts supplying the filling liquid to the circulation circuit to push the cell suspension, which remains in a portion of the circulation circuit from the pump to the inlet port of the reservoir, to flow toward the reservoir by the filling liquid.

## Description

### TECHNICAL FIELD

The present invention relates to a cell suspension treatment apparatus for concentrating cells.

### BACKGROUND ART

In the related art, cell suspension treatment for concentrating cells using a circulation circuit including at least a reservoir, a pump, and a hollow fiber membrane filter has been performed (refer to, for example, Patent Document 1). As the concentration treatment proceeds, the concentration of cells in the cell suspension in the reservoir is increased. Then, when the concentration of the cell suspension in the reservoir reaches a predetermined concentration, the cell concentration treatment is completed.

### PRIOR ART DOCUMENT

### PATENT DOCUMENT

Patent Document 1: J Japanese Unexamined Patent Application Publication No.2015-42167 A

### SUMMARY OF THE INVENTION

### PROBLEMS TO BE SOLVED BY THE INVENTION

Incidentally, when the concentration treatment is completed, many cells are present in the reservoir, but cells remain also in the circulation circuit.

Therefore, an object of the present invention is to collect cells remaining in a circulation circuit after a concentration treatment is completed, in the concentration treatment of cells using the circulation circuit including at least a reservoir, a pump, and a hollow fiber membrane filter.

### MEANS FOR SOLVING THE PROBLEMS

In order to solve the technical problem, according to an aspect of the present invention, there is provided a cell suspension treatment apparatus that performs a concentration treatment on a cell suspension, and the cell suspension treatment apparatus includes a circulation circuit in which the cell suspension is circulated; and a first filling liquid supply source that supplies a filling liquid to the circulation circuit, in which the circulation circuit includes a hollow fiber membrane filter that filters and concentrates the cell suspension, a reservoir that includes an inlet port and an outlet port, and stores the cell suspension, a pump that is provided on a downstream side of the reservoir in a circulation direction of the cell suspension and on an upstream side of the hollow fiber membrane filter in the circulation direction, and circulates the cell suspension, and a first valve that is provided on the downstream side of the reservoir in the circulation direction and on an upstream side of the pump in the circulation direction, the first filling liquid supply source is connected to a portion of the circulation circuit between the first valve and the pump, and after the concentrated cell suspension is stored in the reservoir, in a state where the first valve is closed and the pump is driven in a forward direction, the first filling liquid supply source starts supplying the filling liquid to the circulation circuit to push the cell suspension, which remains in a portion of the circulation circuit from the pump to the inlet port of the reservoir, to flow toward the reservoir in the circulation direction by the filling liquid.

Further, according to another aspect of the present invention, there is provided a cell suspension treatment apparatus that treats a cell suspension, and the cell suspension treatment apparatus includes a pillar portion; an arm portion that extends from the pillar portion in a lateral direction; a cell treatment circuit that is provided on the pillar portion, and includes a circulation circuit that includes at least a storage bag that stores the cell suspension, a pump, and a hollow fiber membrane filter, and concentrates the cell suspension; and a plurality of hooks which are provided on the arm portion in a state of being arranged side by side in an extending direction of the arm portion, and by which the storage bag and a plurality of bag included in the cell treatment circuit and connected to the circulation circuit are suspended.

Furthermore, according to still another aspect of the present invention, there is provided a cell suspension treatment apparatus that perform a concentration treatment and a washing treatment of cells, and the cell suspension treatment apparatus includes a circulation circuit that includes a reservoir that stores a cell suspension, a hollow fiber membrane filter that filters and concentrates the cell suspension, a first connection tube that connects an outlet port of the reservoir to an inlet port of the hollow fiber membrane filter, and a second connection tube that connects an outlet port of the hollow fiber membrane filter to an inlet port of the reservoir; a first branch tube that branches from the first connection tube and is connected to a cell suspension supply source; a second branch tube that branches from the second connection tube and is connected to a replacement liquid supply source; a roller pump that is provided in the first or second connection tube; a first pinch device that pinches the first branch tube; and a second pinch device that pinches the second branch tube, in which when the cell suspension is supplied from the cell suspension supply source to the circulation circuit, the second pinch device pinches and closes the second branch tube, and when the replacement liquid is supplied from the replacement liquid supply source to the circulation circuit, the first pinch device pinches and closes the first branch tube.

### EFFECTS OF THE INVENTION

According to the present invention, it is possible to collect cells remaining in a circulation circuit after a concentration treatment is completed, in the concentration treatment of cells using the circulation circuit including at least a reservoir, a pump, and a hollow fiber membrane filter.

### BRIEF DESCRIPTION OF THE DRAWINGS

FIG. 1 is a front perspective view of a cell suspension treatment apparatus according to an embodiment of the present invention.
FIG. 2 is a front perspective view of the cell suspension treatment apparatus viewed from a different viewpoint.
FIG. 3 is a rear perspective view of the cell suspension treatment apparaus.
FIG. 4 is a rear perspective view of the cell suspension treatment apparatus viewed from a different viewpoint.
FIG. 5 is a front view of the cell suspension treatment apparatus.
FIG. 6 is a rear view of the cell suspension treatment apparatus.
FIG. 7 is a right side view of the cell suspension treatment apparatus.
FIG. 8 is a left side view of the cell suspension treatment apparatus.
FIG. 9 is a plan view of the cell suspension treatment apparatus.
FIG. 10 is a front view of the cell suspension treatment apparatus in a state where an example of a cell concentration and washing circuit is set.
FIG. 11 is a schematic configuration diagram of an example cell of the cell concentration and washing circuit.
FIG. 12 is a perspective view of a part of the cell suspension treatment apparatus illustrating a plurality of hooks.
FIG. 13 is a block diagram illustrating a control system of the cell suspension treatment apparatus.
FIG. 14A is a diagram for describing a first treatment step in an example of a cell suspension treatment.
FIG. 14B is a diagram for describing a second treatment step subsequent to the first treatment step.
FIG. 14C is a diagram for describing a third treatment step subsequent to the second treatment step.
FIG. 14D is a diagram illustrating a fourth treatment step subsequent to the third treatment step.
FIG. 14E is a diagram for describing a fifth treatment step subsequent to the fourth treatment step.
FIG. 14F is a diagram illustrating a sixth treatment step subsequent to the fifth treatment step.
FIG. 14G is a diagram for describing a seventh treatment step subsequent to the sixth treatment step.
FIG. 14H is a diagram illustrating an eighth treatment step subsequent to the seventh treatment step.
FIG. 14I is a diagram for describing a ninth treatment step subsequent to the eighth treatment step.
FIG. 14J is a diagram illustrating a tenth treatment step subsequent to the ninth treatment step.
FIG. 14K is a diagram for describing an eleventh treatment step subsequent to the tenth treatment step.
FIG. 15 is a perspective view illustrating an example of a pinch device.
FIG. 16 is a graph illustrating pressure fluctuations of the cell suspension during supply to the circulation circuit in a case where an accumulator is present (example) and in a case where an accumulator is not present (comparative example).
FIG. 17 is a graph illustrating pressure fluctuations of the cell suspension during cell washing in a case where an accumulator is present (example) and in a case where an accumulator is not present (comparative example).

### MODE(S) FOR CARRYING OUT THE INVENTION

A cell suspension treatment apparatus according to an aspect of the present invention is a cell suspension treatment apparatus that performs a concentration treatment on a cell suspension, and the cell suspension treatment apparatus includes a circulation circuit in which the cell suspension is circulated; and a first filling liquid supply source that supplies a filling liquid to the circulation circuit, in which the circulation circuit includes a hollow fiber membrane filter that filters and concentrates the cell suspension, a reservoir that includes an inlet port and an outlet port, and stores the cell suspension, a pump that is provided on a downstream side of the reservoir in a circulation direction of the cell suspension and on an upstream side of the hollow fiber membrane filter in the circulation direction, and circulates the cell suspension, and a first valve that is provided on the downstream side of the reservoir in the circulation direction and on an upstream side of the pump in the circulation direction, the first filling liquid supply source is connected to a portion of the circulation circuit between the first valve and the pump, and after the concentrated cell suspension is stored in the reservoir, in a state where the first valve is closed and the pump is driven in a forward direction, the first filling liquid supply source starts supplying the filling liquid to the circulation circuit to push the cell suspension, which remains in a portion of the circulation circuit from the pump to the inlet port of the reservoir, to flow toward the reservoir in the circulation direction by the filling liquid.

According to the aspect, it is possible to collect cells remaining in the circulation circuit after the concentration treatment is completed, in the concentration treatment of cells using the circulation circuit including at least the reservoir, the pump, and the hollow fiber membrane filter.

The cell suspension treatment apparatus may further include a first optical sensor that is provided near the inlet port of the reservoir, and detects a color and/or turbidity of the cell suspension, and in this case, when the first optical sensor detects a change in color and/or turbidity of the cell suspension, the pump is stopped, and the first filling liquid supply source stops supplying the filling liquid to the circulation circuit.

The first filling liquid supply source may be a filling liquid supply source for priming the circulation circuit and the hollow fiber membrane filter.

The cell suspension treatment apparatus may further include a second filling liquid supply source that supplies a filling liquid to the circulation circuit; and a second valve that is provided on a downstream side of the hollow fiber membrane filter in the circulation direction and on an upstream side of the reservoir in the circulation direction. In this case, the second filling liquid supply source is connected to a portion of the circulation circuit between the pump and the second valve, and after the concentrated cell suspension is stored in the reservoir, in a state where the second valve is closed and the pump is driven in a reverse direction, the second filling liquid supply source starts supplying the filling liquid to the circulation circuit to push the cell suspension, which remains in a portion of the circulation circuit from the outlet port of the reservoir to the pump, to flow toward the reservoir in a direction opposite to the circulation direction by the filling liquid.

The cell suspension treatment apparatus may further include a second optical sensor that is provided near the outlet port of the reservoir, and detects a color and/or turbidity of the cell suspension, and in this case, when the second optical sensor detects a change in color and/or turbidity of the cell suspension, the pump is stopped, and the second filling liquid supply source stops supplying the filling liquid to the circulation circuit.

The second filling liquid supply source may be a filling liquid supply source for priming the hollow fiber membrane filter positioned between the pump and the second valve by supplying the filling liquid into the hollow fiber membrane filter.

A cell suspension treatment apparatus according to another aspect of the present invention is a cell suspension treatment apparatus that treats a cell suspension, and the cell suspension treatment apparatus includes a pillar portion; an arm portion that extends from the pillar portion in a lateral direction; a cell treatment circuit that is provided on the pillar portion, and includes a circulation circuit that includes at least a storage bag that stores the cell suspension, a pump, and a hollow fiber membrane filter, and concentrates the cell suspension; and a plurality of hooks which are provided on the arm portion in a state of being arranged side by side in an extending direction of the arm portion, and by which the storage bag and a plurality of bag included in the cell treatment circuit and connected to the circulation circuit are suspended.

According to the aspect, it is possible to appropriately dispose a plurality of necessary container with respect to the circulation circuit while making an installation space compact, in the cell suspension treatment using the circulation circuit including at least the reservoir, the pump, and the hollow fiber membrane filter.

At least one of the plurality of hooks may be movable in the extending direction of the arm portion.

At least one of the plurality of hooks may be provided on the arm portion via a weight sensor.

The cell suspension treatment apparatus may further include a drain pan that is disposed immediately below the bag suspended by each of the plurality of hooks.

The cell treatment circuit may be provided on a front surface of the pillar portion.

The cell suspension treatment apparatus may further include a plurality of casters; and a handle for a user to operate when the user moves the cell suspension treatment apparatus via the plurality of casters. In this case, the handle may function as a guard that extends to a side of a tip end of the arm portion to avoid contact between the user and the bag on an outermost side.

The plurality of bags may include a filling liquid bag that supplies a filling liquid for priming the circulation circuit to the circulation circuit, a waste liquid bag that collects the filling liquid after priming, and a replacement liquid bag that supplies a replacement liquid for washing cells of the concentrated cell suspension stored in the storage bag, to the circulation circuit.

A cell suspension treatment apparatus according to still another aspect of the present invention is a cell suspension treatment apparatus that perform a concentration treatment and a washing treatment of cells, and the cell suspension treatment apparatus includes a circulation circuit that includes a reservoir that stores a cell suspension, a hollow fiber membrane filter that filters and concentrates the cell suspension, a first connection tube that connects an outlet port of the reservoir to an inlet port of the hollow fiber membrane filter, and a second connection tube that connects an outlet port of the hollow fiber membrane filter to an inlet port of the reservoir; a first branch tube that branches from the first connection tube and is connected to a cell suspension supply source; a second branch tube that branches from the second connection tube and is connected to a replacement liquid supply source; a roller pump that is provided in the first or second connection tube; a first pinch device that pinches the first branch tube; and a second pinch device that pinches the second branch tube, in which when the cell suspension is supplied from the cell suspension supply source to the circulation circuit, the second pinch device pinches and closes the second branch tube, and when the replacement liquid is supplied from the replacement liquid supply source to the circulation circuit, the first pinch device pinches and closes the first branch tube.

According to the aspect, in the concentration treatment and the washing treatment of cells using the circulation circuit including at least the reservoir, the pump, and the hollow fiber membrane filter, the pulsation of the cell suspension flowing in the circulation circuit can be suppressed.

At least one of the first and second branch tubes may extend from a branch point of the first or second branch tube in an orthogonal direction with respect to a flow direction of the cell suspension passing through the branch point.

At least one of the first and second branch tubes may extend from a branch point of the first or second branch tube in an obliquely rearward direction with respect to a flow direction of the cell suspension passing through the branch point.

At least one of the first and second branch tubes may extend from a branch point of the first or second branch tube in an upward direction with respect to a flow direction of the cell suspension passing through the branch point.

Each of the first and second pinch devices may be a pinch valve or forceps.

The first and second branch tubes may include a plurality of indicators indicating a plurality of pinch positions.

Hereinafter, embodiments of the present invention will be described with reference to the drawings. However, unnecessarily detailed description may be omitted. For example, a detailed description of a well-known matter and a repeated description of substantially the same configuration may be omitted. This is to avoid unnecessary redundancy of the following description and to facilitate understanding of those skilled in the art.

Note that the inventor(s) provides the accompanying drawings and the following description in order for those skilled in the art to fully understand the present invention, and does not intend to limit the subject matter described in the claims by the accompanying drawings and the following description.

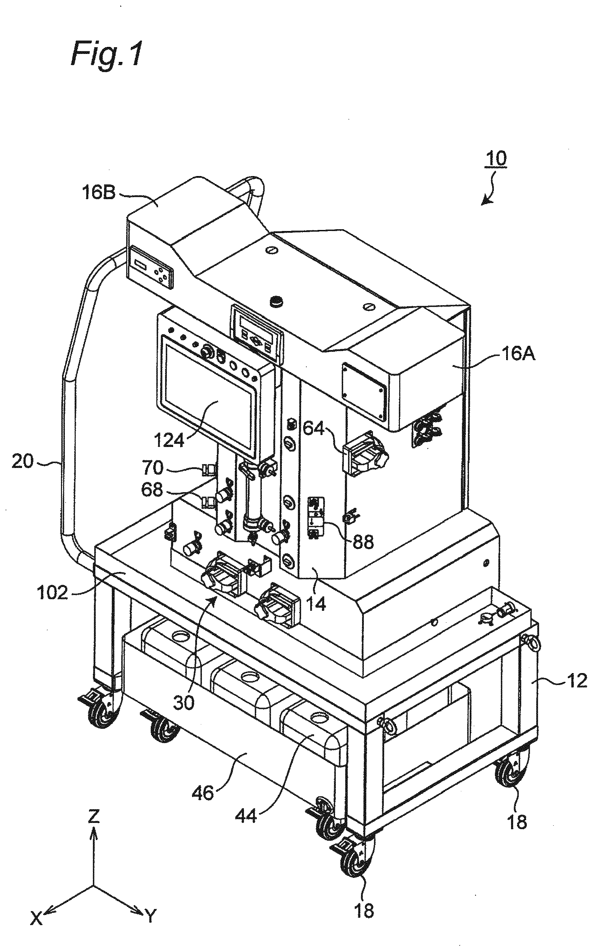

FIGS. 1 to 9 illustrate the appearance of a cell suspension treatment apparatus according to an embodiment of the present invention.

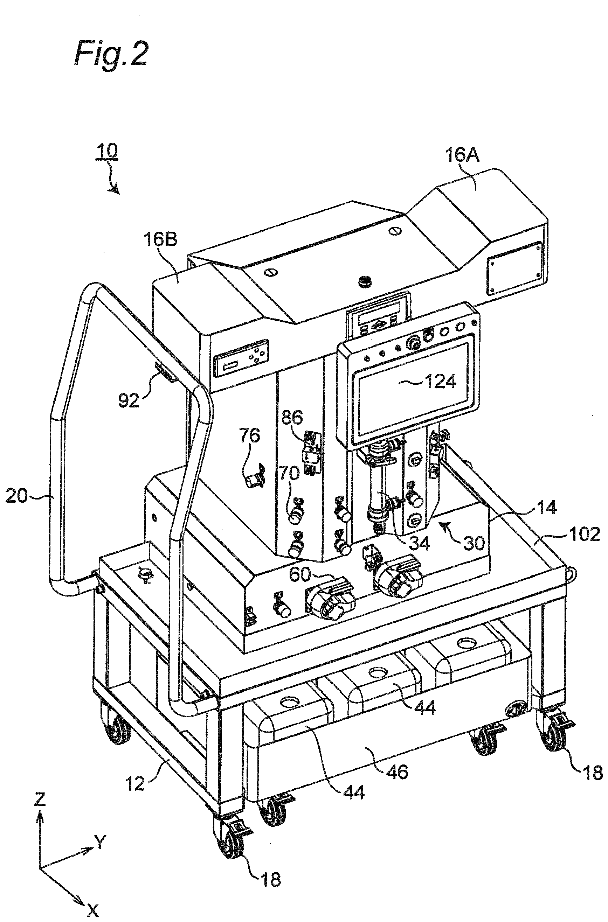

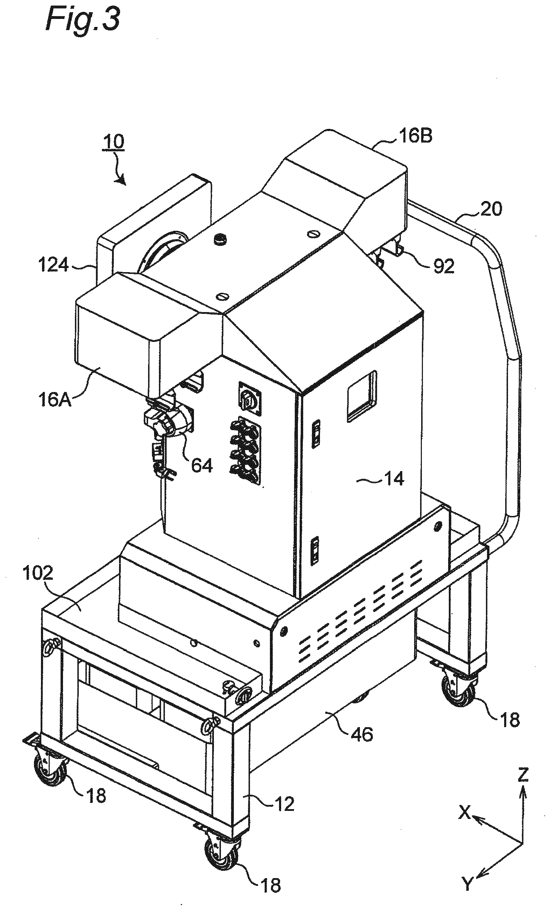

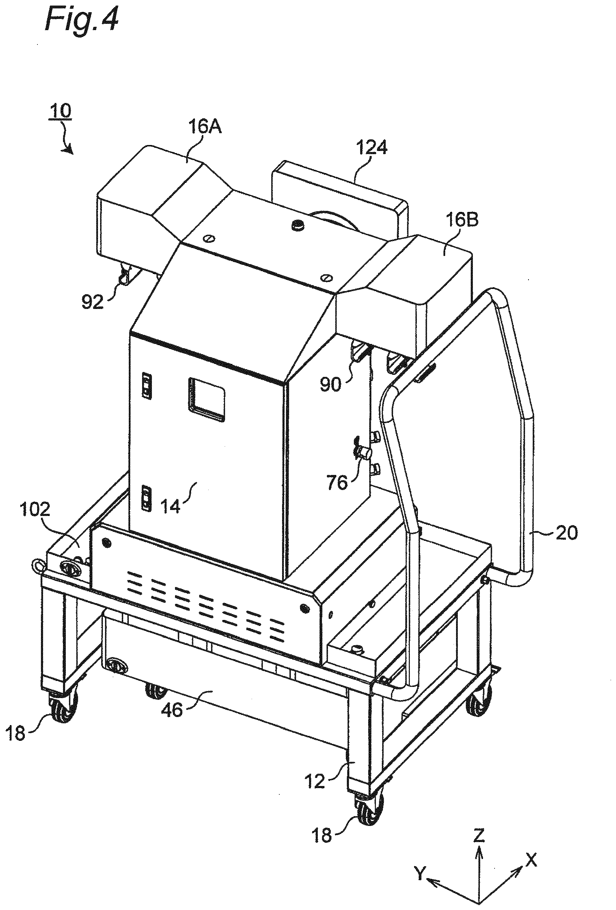

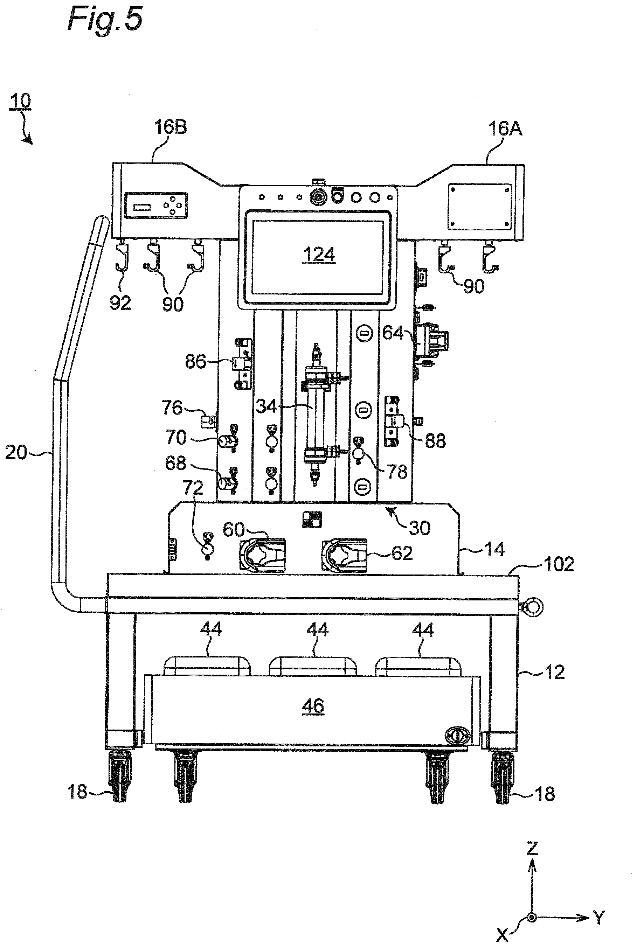

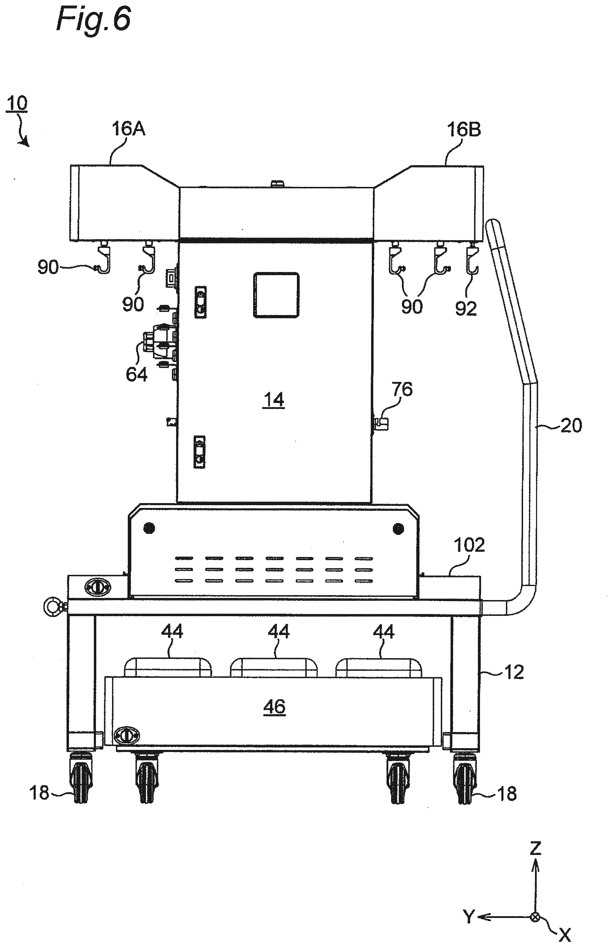

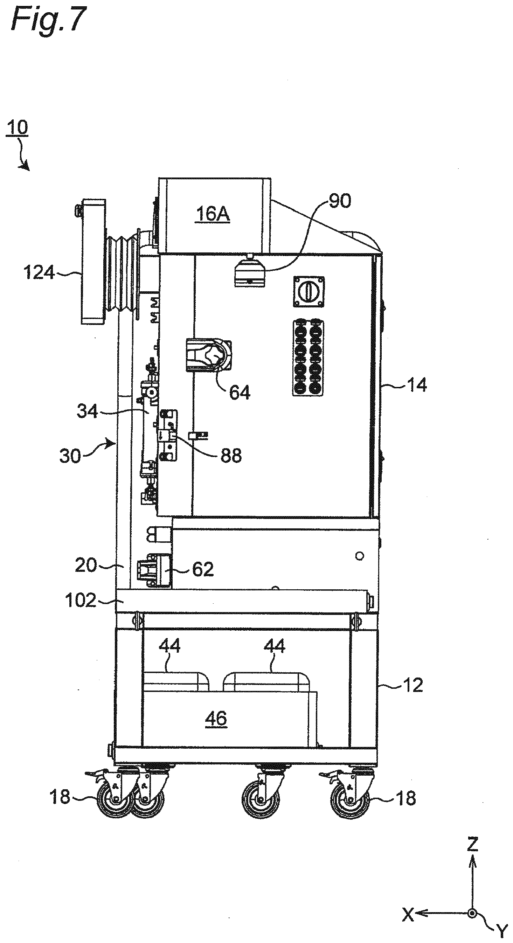

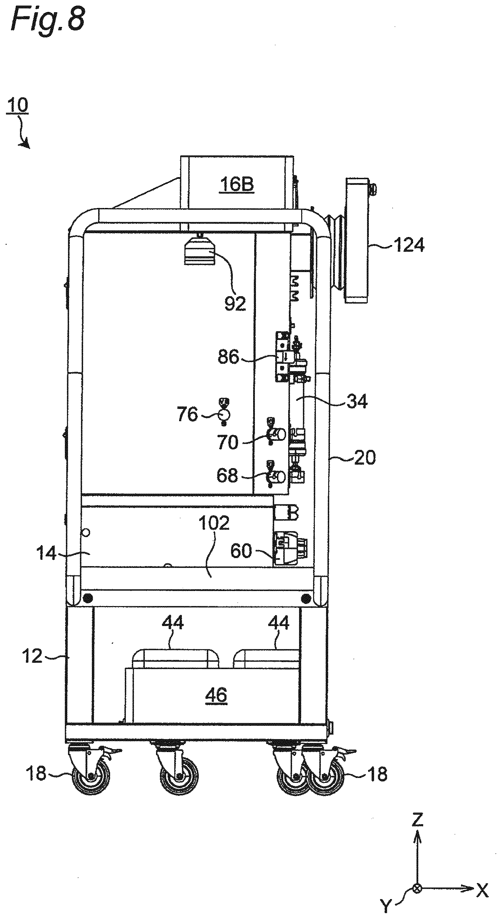

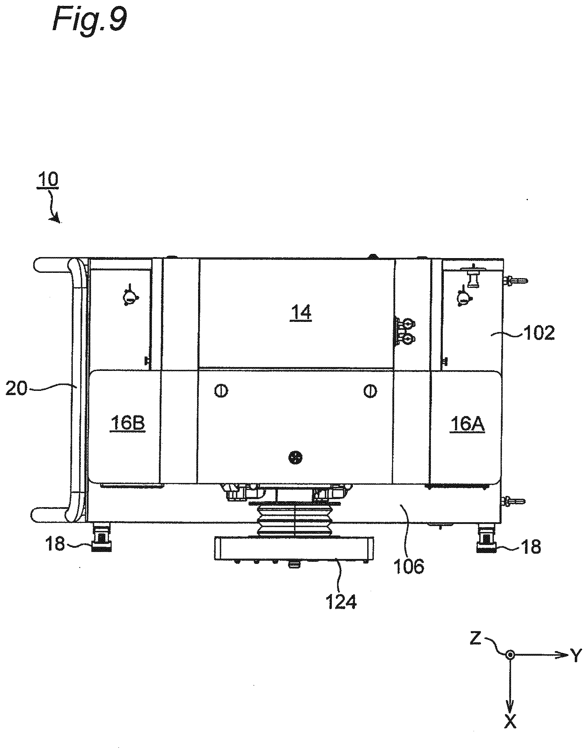

Specifically, FIG. 1 is a front perspective view of the cell suspension treatment apparatus. FIG. 2 is a front perspective view of the cell suspension treatment apparatus viewed from a different viewpoint. FIG. 3 is a rear perspective view of the cell suspension treatment apparatus. FIG. 4 is a rear perspective view of the cell suspension treatment apparatus viewed from a different viewpoint. FIG. 5 is a front view of the cell suspension treatment apparatus. FIG. 6 is a rear view of the cell suspension treatment apparatus. FIG. 7 is a right side view of the cell suspension treatment apparatus. FIG. 8 is a left side view of the cell suspension treatment apparatus. FIG. 9 is a plan view of the cell suspension treatment apparatus.

Note that an X-Y-Z orthogonal coordinate system is illustrated in the drawings, but this is for facilitating understanding of the embodiments of the invention and does not limit the invention. The X-axis direction is a front-rear direction of the cell suspension treatment apparatus, the Y-axis direction is a left-right direction, and the Z-axis direction is a height direction. The expression "left-right" is based on a front view of the cell suspension treatment apparatus.

A cell suspension treatment apparatus 10 according to the present embodiment illustrated in FIGS. 1 to 9 is an apparatus for performing a concentration treatment and a washing treatment on cells as the cell suspension treatment. Note that FIGS. 1 to 9 illustrate the cell suspension treatment apparatus 10 in a state before a bag, a tube, or the like used for the cell suspension treatment is attached, that is, the main body of the cell suspension treatment apparatus 10. The "cell suspension" refers to a suspension of cells such as platelets and megakaryocytes in a liquid medium. The embodiment of the present invention does not limit the cells and the medium.

In addition, the "concentration" treatment refers to a treatment in which a cell suspension is filtered to remove a medium component such that the concentration of the cells in the cell suspension is increased. In addition, the "washing" treatment refers to a treatment of substituting a medium component of the concentrated cell suspension with a replacement liquid such as physiological saline, physiological saline with a buffering effect, Ringer's solution such as bicarbonate Ringer's solution (bicanate infusion solution; manufactured by Otsuka Pharmaceutical Factory, Inc.), a solution obtained by adding, to the bicarbonate Ringer's solution, a blood preservation solution (ACD-A solution; manufactured by Terumo Corporation), ACD-A solution and albumin such as human serum albumin preparation (HAS; manufactured by CSL Behring), or ACD-A solution, human serum albumin preparation, and an antioxidant.

As illustrated in FIGS. 1 to 9, the cell suspension treatment apparatus 10 according to the present embodiment includes a base portion 12, a pillar portion 14 provided on the base portion 12, and first and second arm portions 16A and 16B provided on the pillar portion 14.

The base portion 12 of the cell suspension treatment apparatus 10 is a desk-shaped structure, and includes a plurality of casters 18. The plurality of casters 18 allow the entire cell suspension treatment apparatus 10 to be movable. In addition, a handle 20 for a user to operate the cell suspension treatment apparatus 10 that is moved through the plurality of casters 18 is provided on a left side portion of the base portion 12. As a result, it is possible to easily change the layout of the cell suspension treatment apparatus 10.

The pillar portion 14 of the cell suspension treatment apparatus 10 is a pillar-shaped structure that is provided on the base portion 12, and extends in an upward direction (Z-axis direction) from the base portion 12. As will be described in detail later, a cell treatment circuit for the treatment of cells, specifically, a cell concentration and washing circuit for the concentration treatment and the washing treatment of cells is provided on the front surface of the pillar portion 14.

The first and second arm portions 16A and 16B of the cell suspension treatment apparatus 10 extend from the upper portion of the pillar portion 14 in a lateral direction, in the horizontal direction (Y-axis direction) in the present embodiment. The first arm portion 16A extends in the right direction, and the second arm portion 16B extends in the left direction. Although details will be described later, the first and second arm portions 16A and 16B are configured such that a plurality of bags to be used for the concentration treatment and the washing treatment of cells can be suspended.

Hereinafter, the cell concentration and washing circuit for the concentration treatment and the washing treatment of cells will be described.

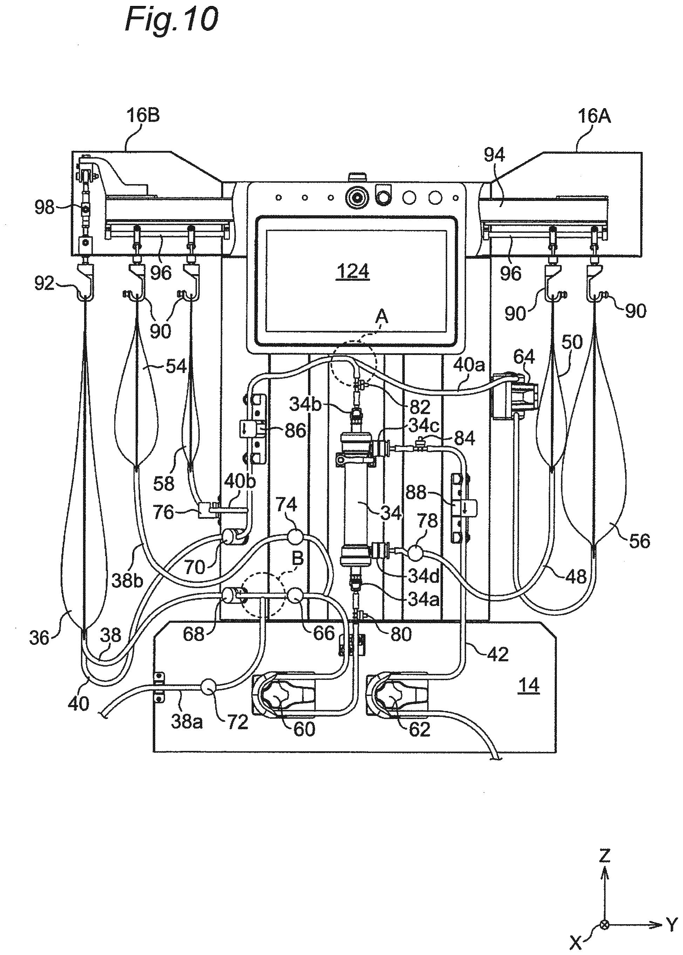

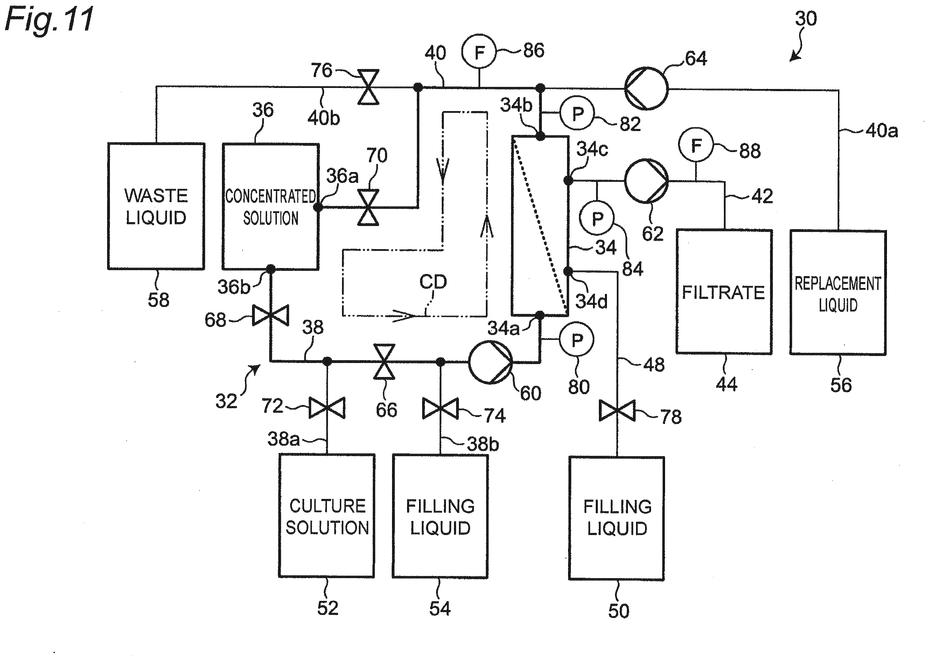

FIG. 10 is a front view of the cell suspension treatment apparatus in a state where an example of the cell concentration and washing circuit is set. FIG. 11 is a schematic configuration diagram of an example cell of the cell concentration and washing circuit.

As illustrated in FIG. 11, a cell concentration and washing circuit 30 includes a circulation circuit 32 in which the cell suspension is circulated during the concentration treatment and the washing treatment.

The circulation circuit 32 includes a hollow fiber membrane filter 34 that filters the cell suspension, a storage bag 36 that stores the concentrated cell suspension (concentrated solution), a first connection tube 38 that connects an outlet port 36b of the storage bag 36 to an inlet port 34a of the hollow fiber membrane filter 34, and a second connection tube 40 that connects an outlet port 34b of the hollow fiber membrane filter 34 to an inlet port 36a of the storage bag 36.

The hollow fiber membrane filter 34 is a device that filters and concentrates the cell suspension circulated in the circulation circuit 32, and is exchangeably attached to the front surface of the pillar portion 14 of the cell suspension treatment apparatus 10. The hollow fiber membrane filter 34 includes the inlet port 34a into which the cell suspension flows, the outlet port 34b through which the cell suspension concentrated by filtration flows out, and a filtrate discharge port 34c through which filtrate generated by filtration is discharged. The filtrate discharge port 34c of the hollow fiber membrane filter 34 is connected to a filtrate tank 44 through a connection tube 42.

As illustrated in FIG. 1, the filtrate tank 44 is mounted on a truck 46 disposed in the base portion 12 of the cell suspension treatment apparatus 10. By moving the truck 46, the filtrate tank 44 containing the filtrate can be moved to an appropriate place.

Further, in the present embodiment, the hollow fiber membrane filter 34 includes an introduction port 34d through which a filling liquid for priming is introduced to the inside of the hollow fiber membrane filter. The introduction port 34d is connected to a filling liquid bag 50 that supplies the filling liquid for priming, through a connection tube 48.

The filling liquid bag 50 is a container which contains a filling liquid such as physiological saline, physiological saline with a buffering effect, Ringer's solution such as bicarbonate Ringer's solution (bicanate infusion solution; manufactured by Otsuka Pharmaceutical Factory, Inc.), a solution obtained by adding, to the bicarbonate Ringer's solution, a blood preservation solution (ACD-A solution; manufactured by Terumo Corporation), ACD-A solution and albumin such as human serum albumin preparation (HAS; manufactured by CSL Behring), or ACD-A solution, human serum albumin preparation, and an antioxidant, and the filling liquid bag 50 is made of, for example, a resin material, and has flexibility. In addition, although details will be described later, the filling liquid bag 50 is suspended from the first arm portion 16A.

The storage bag 36 is a container (reservoir) that stores a cell suspension during the concentration treatment and the washing treatment of cells, is made of, for example, a resin material, and has flexibility. Further, although details will be described later, the storage bag 36 is suspended from the second arm portion 16B. Furthermore, the storage bag 36 includes the inlet port 36a into which the cell suspension from the hollow fiber membrane filter 34 flows, and the outlet port 36b through which the cell suspension flows out toward the hollow fiber membrane filter 34.

The first connection tube 38 is a flexible tube made of a transparent resin material, and connects the outlet port 36b of the storage bag 36 to the inlet port 34a of the hollow fiber membrane filter 34.

The first connection tube 38 branches at two points, that is, two branch tubes 38a and 38b are connected. One branch tube 38a is connected to a container which contains a culture solution that has been cultured, that is, a culture solution tank (cell suspension supply source) 52 which supplies the cell suspension stored in the storage bag 36 to the circulation circuit 32, which will be described in detail later. The culture solution tank 52 is disposed outside the cell suspension treatment apparatus 10. The other branch tube 38b is connected to a filling liquid bag 54 that supplies a filling liquid for priming to the circulation circuit 32, which will be described in detail later.

The filling liquid bag 54 is a container which contains a filling liquid such as physiological saline, physiological saline with a buffering effect, Ringer's solution such as bicarbonate Ringer's solution (bicanate infusion solution; manufactured by Otsuka Pharmaceutical Factory, Inc.), a solution obtained by adding, to the bicarbonate Ringer's solution, a blood preservation solution (ACD-A solution; manufactured by Terumo Corporation), ACD-A solution and albumin such as human serum albumin preparation (HAS; manufactured by CSL Behring), or ACD-A solution, human serum albumin preparation, and an antioxidant, and the filling liquid bag 54 is made of, for example, a resin material, and has flexibility. In addition, although details will be described later, the filling liquid bag 54 is suspended from the second arm portion 16B.

Similarly to the first connection tube 38, the second connection tube 40 is a flexible tube made of a transparent resin material, and connects the outlet port 34b of the hollow fiber membrane filter 34 to the inlet port 36a of the storage bag 36.

The second connection tube 40 branches at two points, that is, two branch tubes 40a and 40b are connected. One branch tube 40a is connected to a replacement liquid bag (replacement liquid supply source) 56 that supplies a replacement liquid for washing the cells in the concentrated cell suspension, that is, a replacement liquid for replacing the medium component of the cell suspension, to the circulation circuit 32. The other branch tube 40b is connected to a waste liquid bag 58 that collects the filling liquid after priming, which will be described in detail later.

The replacement liquid bag 56 is a container which contains a replacement liquid such as physiological saline, physiological saline with a buffering effect, Ringer's solution such as bicarbonate Ringer's solution (bicanate infusion solution; manufactured by Otsuka Pharmaceutical Factory, Inc.), a solution obtained by adding, to the bicarbonate Ringer's solution, a blood preservation solution (ACD-A solution; manufactured by Terumo Corporation), ACD-A solution and albumin such as human serum albumin preparation (HAS; manufactured by CSL Behring), or ACD-A solution, human serum albumin preparation, and an antioxidant, and the replacement liquid bag 56 is made of, for example, a resin material, and has flexibility. In addition, although details will be described later, the replacement liquid bag 56 is suspended from the first arm portion 16A.

The waste liquid bag 58 is a container that collects the filling liquid after priming, is made of, for example, a resin material, and has flexibility. In addition, although details will be described later, the waste liquid bag 58 is suspended from the second arm portion 16B.

The cell concentration and washing circuit 30 including the circulation circuit 32 includes a plurality of pumps 60 to 64, a plurality of valves 66 to 78, a plurality of pressure sensors 80 to 84, and a plurality of flow rate sensors 86 to 88 in order to perform the concentration treatment and the washing treatment of cells.

The circulation circuit 32 of the cell concentration and washing circuit 30 is provided with the pump 60 and three valves 66 to 70.

The pump 60 in the circulation circuit 32 is a pump mainly for circulating the cell suspension in the circulation circuit 32, for example, a roller pump. The pump 60 is arranged on the downstream side of the storage bag 36 in a circulation direction CD of the cell suspension, and on the upstream side of the hollow fiber membrane filter 34 in the circulation direction CD, that is, is provided in the first connection tube 38. The pump 60 is attached to the front surface of the pillar portion 14 of the cell suspension treatment apparatus 10.

The three valves 66 to 70 in the circulation circuit 32 are closed pinch valves, for example, pinching the first and second connection tubes 38 and 40. In the three valves, the two valves 66 and 68 are provided in the first connection tube 38. Specifically, the valve 66 is disposed between the branch points of the two branch tubes 38a and 38b, and the valve 68 is disposed between the storage bag 36 and the branch point of the branch tube 38a. The remaining valve 70 is provided in the second connection tube 40, and is specifically disposed between the branch point of the branch tube 40b and the storage bag 36. In addition, these three valves 66 to 70 are attached to the front surface of the pillar portion 14 of the cell suspension treatment apparatus 10.

The pump 62 provided outside the circulation circuit 32 is a pump for sending the filtrate of the hollow fiber membrane filter 34 to the filtrate tank 44, and is, for example, a roller pump. The pump 62 is provided in the connection tube 42 that connects the filtrate discharge port 34c of the hollow fiber membrane filter 34 to the filtrate tank 44. The pump 62 is attached to the front surface of the pillar portion 14 of the cell suspension treatment apparatus 10.

The pump 64 provided outside the circulation circuit 32 is a pump for sending the replacement liquid in the replacement liquid bag 56 toward the circulation circuit 32, and is, for example, a roller pump. The pump 64 is provided in the branch tube 40a connected to the second connection tube 40. The pump 64 is attached to a right side surface of the pillar portion 14 of the cell suspension treatment apparatus 10.

The valve 72 provided outside the circulation circuit 32 is a valve that interrupts the connection between the circulation circuit 32 and the culture solution tank 52, and is, for example, a pinch valve. The valve 72 is provided in the branch tube 38a connected to the first connection tube 38. The valve 72 is attached to the front surface of the pillar portion 14 of the cell suspension treatment apparatus 10.

The valve 74 provided outside the circulation circuit 32 is a valve that interrupts the connection between the circulation circuit 32 and the filling liquid bag 54, and is, for example, a pinch valve. The valve 74 is provided in the branch tube 38b connected to the first connection tube 38. The valve 74 is attached to the front surface of the pillar portion 14 of the cell suspension treatment apparatus 10.

The valve 76 provided outside the circulation circuit 32 is a valve that interrupts the connection between the circulation circuit 32 and the waste liquid bag 58, and is, for example, a pinch valve. The valve 76 is provided in the branch tube 40b connected to the second connection tube 40. The valve 76 is attached to the left side surface of the pillar portion 14 of the cell suspension treatment apparatus 10.

The valve 78 provided outside the circulation circuit 32 is a valve that interrupts the connection between the hollow fiber membrane filter 34 and the filling liquid bag 50, and is, for example, a pinch valve. The valve 78 is provided in the connection tube 48 that connects the introduction port 34d of the hollow fiber membrane filter 34 to the filling liquid bag 50. The valve 78 is attached to the front surface of the pillar portion 14 of the cell suspension treatment apparatus 10.

The pressure sensor 80 is a sensor for detecting a pressure of the cell suspension flowing into the hollow fiber membrane filter 34, and is provided in the first connection tube 38. Specifically, the pressure sensor 80 is disposed between the pump 60 and the hollow fiber membrane filter 34. The pressure sensor 80 is disposed on the front surface of the pillar portion 14 of the cell suspension treatment apparatus 10.

The pressure sensor 82 is a sensor for detecting a pressure of the cell suspension flowing out from the hollow fiber membrane filter 34, and is provided in the second connection tube 40. Specifically, the pressure sensor 82 is disposed between the hollow fiber membrane filter 34 and the branch point of the branch tube 40a. The pressure sensor 82 is disposed on the front surface of the pillar portion 14 of the cell suspension treatment apparatus 10.

The pressure sensor 84 is a sensor for detecting a pressure of the filtrate discharged from the hollow fiber membrane filter 34, and is provided in the connection tube 42 that connects the hollow fiber membrane filter 34 to the filtrate tank 44. Specifically, the pressure sensor 84 is disposed between the hollow fiber membrane filter 34 and the pump 62. The pressure sensor 84 is disposed on the front surface of the pillar portion 14 of the cell suspension treatment apparatus 10.

The flow rate sensor 86 is a sensor, for example, a clamp-on type sensor, for detecting a flow rate of the cell suspension flowing out from the hollow fiber membrane filter 34, that is, a flow rate of the concentrated cell suspension, and is provided in the second connection tube 40. Specifically, the flow rate sensor 86 is disposed between the branch point of the branch tube 40a and the branch point of the branch tube 40b. The flow rate sensor 86 is attached to the front surface of the pillar portion 14 of the cell suspension treatment apparatus 10.

The flow rate sensor 88 is a sensor, for example, a clamp-on type sensor, for detecting a flow rate of the filtrate discharged from the hollow fiber membrane filter, and is provided in the connection tube 42 that connects the hollow fiber membrane filter 34 to the filtrate tank 44. Specifically, the flow rate sensor 88 is disposed between the pump 62 and the filtrate tank 44. The flow rate sensor 88 is attached to the front surface of the pillar portion 14 of the cell suspension treatment apparatus 10.

As illustrated in FIG. 10, the plurality of components of the cell concentration and washing circuit 30 described above are disposed on the front surface of (partially on the side surface of) the pillar portion 14 of the cell suspension treatment apparatus 10. In addition, the replacement liquid bag 56, the filling liquid bag 50, the waste liquid bag 58, the filling liquid bag 54, and the storage bag 36 are provided side by side in the left-right direction (Y-axis direction). As a result, the user can confirm the cell concentration and washing circuit 30 at a glance when positioned to face the front surface of the cell suspension treatment apparatus 10. In addition, from another viewpoint, since the components of the cell concentration and washing circuit 30 and the bags are not present on the rear surface of the pillar portion 14 illustrated in FIG. 6, the cell suspension treatment apparatus 10 can be installed in a state where the rear surface of the pillar portion 14 is close to the wall.

Specifically, as illustrated in FIG. 10, the replacement liquid bag 56, the filling liquid bag 50, the waste liquid bag 58, the filling liquid bag 54, and the storage bag 36 are suspended from the first and second arm portions 16A and 16B. Therefore, the first and second arm portions 16A and 16B include a plurality of hooks 90 and 92 by which the bags are suspended. Two hooks 90 are provided side by side in the extending direction (Y-axis direction) in the first arm portion 16A, and two hooks 90 and one hook 92 are provided side by side in the extending direction (Y-axis direction) in the second arm portion 16B.

In the case of the present embodiment, as illustrated in FIG. 10, a beam 94 extending in the left-right direction (Y-axis direction) is provided in the first and second arm portions 16A and 16B. The beam 94 is provided with a rail 96 that extends in the left-right direction and supports the plurality of hooks 90 such that the plurality of hooks 90 are movable in the left-right direction. As a result, the distance between the bags suspended by the plurality of hooks 90 can be easily adjusted.

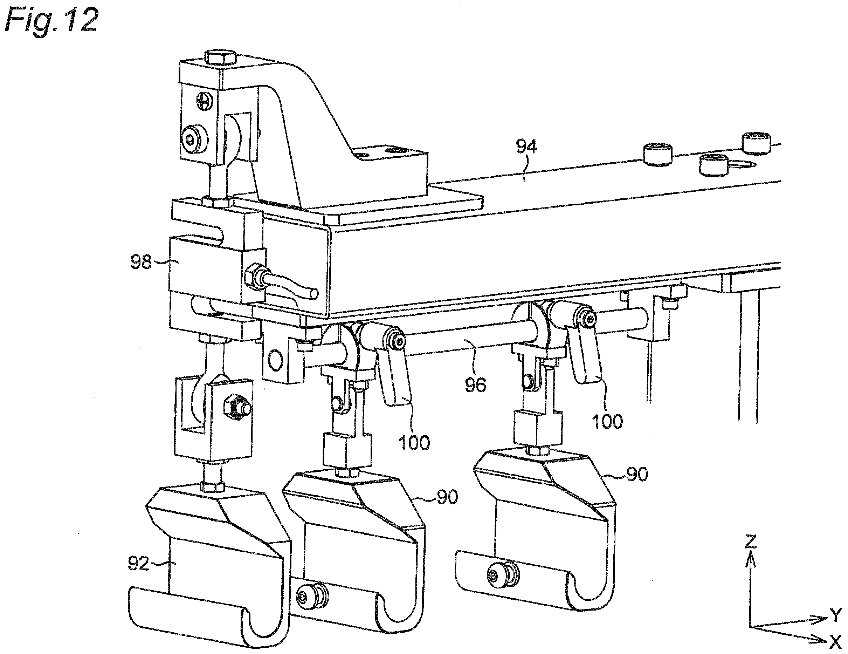

Unlike the other hooks 90, the hook 92 is not movably provided on the beam 94. As illustrated in FIG. 12, the hook 92 is attached to a left end of the beam 94 via a weight sensor 98. This is because the weight of the storage bag 36 suspended by the hook 92 is measured by the weight sensor 98, and the storage bag 36 is heavier and larger than other bags (for example, 50 liters). Similarly to the hook 92, in order to fix the position of each of the plurality of hooks 90, a lock lever 100 for fixing the hook at a predetermined position of the rail 96 is provided in each of the hooks 90.

As illustrated in FIG. 10, the largest storage bag 36 is suspended from the tip end of the second arm portion 16B via the hook 92. That is, the storage bag 36 is positioned on the outermost side. Therefore, there is a possibility that the user on the move comes into contact with the storage bag 36. In order to avoid the contact, for example, as illustrated in FIGS. 1 and 2, the handle 20 extends from the base portion 12 to the side of the tip end of the second arm portion 16B. As a result, the handle 20 is deployed outside the storage bag 36, and the handle 20 functions as a guard that avoids contact between the storage bag 36 and the user.

As described above, since the plurality of bags used for the concentration treatment and the washing treatment of cells are suspended and supported, an installation space (footprint) of the cell suspension treatment apparatus 10 can be made compact (compared to a case where the bag is disposed in a laid state or a case where the plurality of containers containing the cell suspension and the like are not the bags).

As illustrated in FIGS. 1 and 2, a drain pan 102 is disposed below the hooks 90 and 92, that is, below the plurality of bags such as the storage bag 36 suspended by the hooks 90 and 92 and the cell concentration and washing circuit 30. Specifically, the drain pan 102 has a shape (that is, a bracket shape) that surrounds the pillar portion 14 except for the rear surface thereof in plan view (as viewed in the Z-axis direction), and is detachably placed on the base portion 12. As a result, the drain pan 102 can receive the cell suspension leaking from the storage bag 36, the replacement liquid bag 56, or the cell concentration and washing circuit 30. As a result, falling of the leaking cell suspension onto the floor in the room where the cell suspension treatment apparatus 10 is installed is suppressed. Since the drain pan 102 is detachable from the base portion 12 of the cell suspension treatment apparatus 10, the drain pan 102 can be washed at a place different from the installation place of the cell suspension treatment apparatus 10. In addition, if a plurality of drain pans 102 are prepared, the cell suspension treatment apparatus 10 can be used at a high operation rate.

The concentration treatment and the washing treatment of cells using the cell concentration and washing circuit 30 are automatically performed.

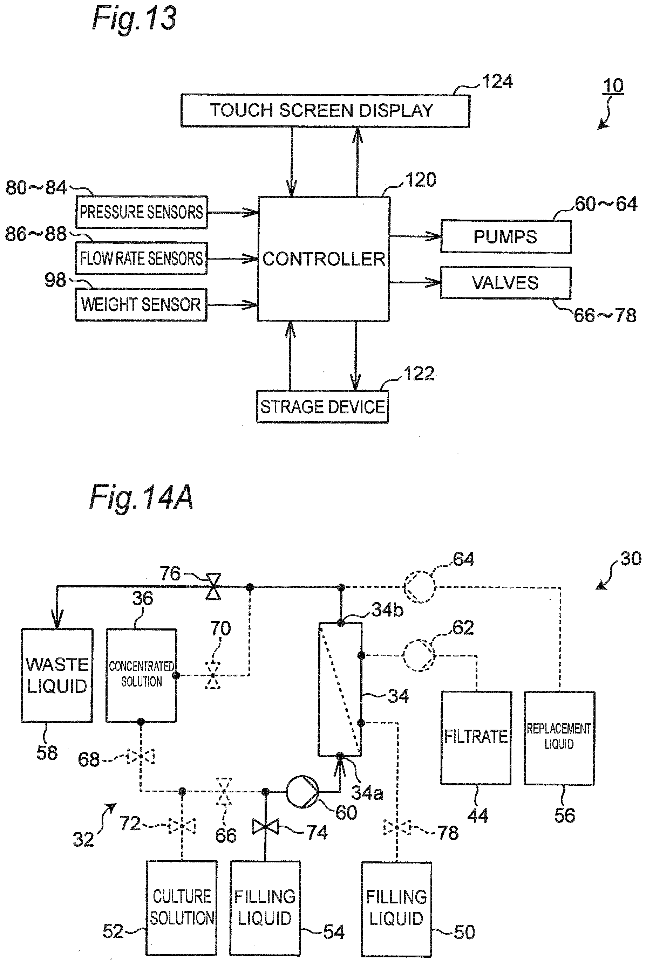

FIG. 13 is a block diagram illustrating a control system of the cell suspension treatment apparatus.

As illustrated in FIG. 13, the cell suspension treatment apparatus 10 has a controller 120 that controls the plurality of pumps 60 to 64 and the plurality of valves 66 to 78. The controller 120 is, for example, a control board on which a CPU is mounted. A storage device 122 and a touch screen display 124 as a user interface are connected to the controller 120. As illustrated in FIG. 10, the touch screen display 124 is attached to the front surface of the pillar portion 14 of the cell suspension treatment apparatus 10.

According to a program stored in the storage device 122 such as a memory or a hard disk, the controller 120 executes control necessary for the concentration treatment and the washing treatment of cells on the plurality of pumps 60 to 64 and the plurality of valves 66 to 78. In addition, the controller 120 controls the plurality of pumps 60 to 64 and the plurality of valves 66 to 78 based on detection results of the plurality of pressure sensors 80 to 84, the plurality of flow rate sensors 86 to 88, and the weight sensor 98. Then, the controller 120 presents information such as the detection results of the plurality of sensors, and the control that is currently being executed (treatment step) to the user via the touch screen display 124.

Hereinafter, the concentration treatment and the washing treatment of cells executed by the controller 120 will be described with reference to FIGS. 14A to 14K.

First, before the concentration treatment and the washing treatment of cells by the controller 120 are started, the setting of the cell concentration and washing circuit 30 is performed by the user as a preliminary preparation.

As the preliminary preparation, as illustrated in FIG. 10, the plurality of connection tubes 38, 40, 42, and 48 and the plurality of pressure sensors 80 to 84 are attached to the plurality of pumps 60 to 64, the plurality of valves 66 to 78, and the plurality of flow rate sensors 86 and 88 on the pillar portion 14 of the cell suspension treatment apparatus 10 by the user. Since the plurality of connection tubes and pressure sensor are used singly, the connection tubes and the pressure sensors are replaced each time the cell treatment is performed.

Next, as illustrated in FIG. 10, the filling liquid bag 50 and the replacement liquid bag 56 are suspended from the first arm portion 16A via the hooks 90 by the user. In addition, the waste liquid bag 58, the filling liquid bag 54, and the storage bag 36 are suspended from the second arm portion 16B via the hooks 90 and 92. At this time, the storage bag 36 and the waste liquid bag 58 are empty.

Subsequently, as illustrated in FIGS. 10 and 11, the plurality of bags 36, 50, 54, 56, and 58 are connected to the circulation circuit 32 by the user. In addition, the branch tube 38a connected to the first connection tube 38 is connected to the culture solution tank 52 located outside the cell suspension treatment apparatus 10. Further, the connection tube 42 connected to the filtrate discharge port 34c of the hollow fiber membrane filter 34 is connected to the filtrate tank 44.

Then, the user sets conditions relating to the concentration treatment and the washing treatment of cells, for example, the rotation speed of the pump 60 to 64, the concentration time, the washing time, and the like via the touch screen display 124.

When the preliminary preparation is completed and the user inputs a start instruction to touch screen display 124, the controller 120 executes, as a first treatment step, a treatment step of washing the inside of the hollow fiber membrane in hollow fiber membrane filter 34. Therefore, as illustrated in FIG. 14A, the controller 120 opens only the valves 74 and 76, and activates only the pump 60. In the drawing, the pump indicated by the broken line is in a stopped state, and the valve indicated by the broken line is in a closed state.

During the execution of the first treatment step, the filling liquid in the filling liquid bag 54 flows into the hollow fiber membrane filter 34 through the inlet port 34a, and the filling liquid flowing out from the outlet port 34b of the hollow fiber membrane filter 34 enters the waste liquid bag 58. Thus, the inside of the hollow fiber membrane is washed.

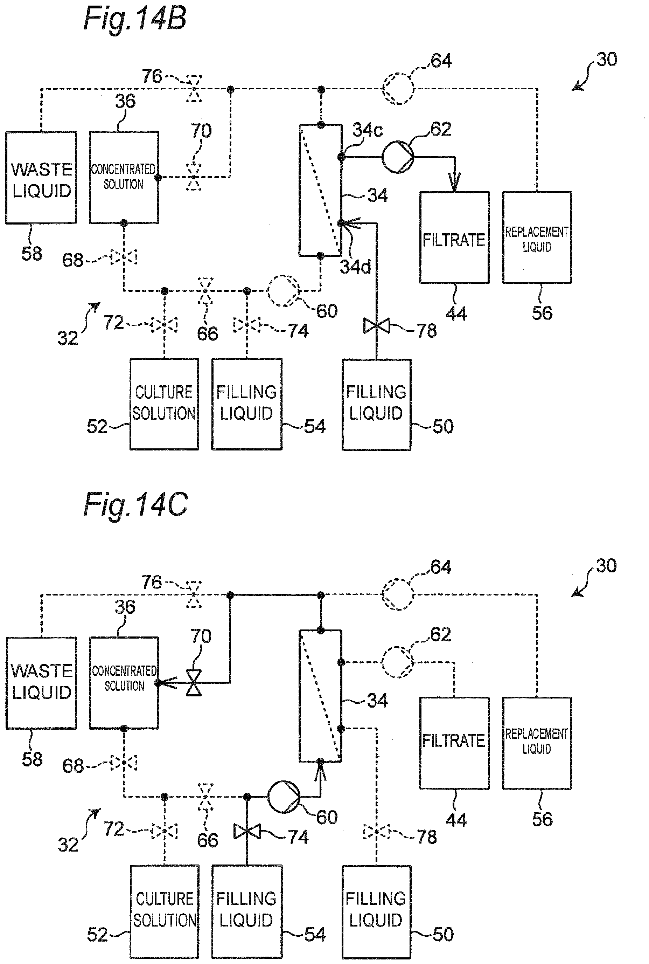

Next, as a second treatment step, the controller 120 executes a treatment step of washing a space outside the hollow fiber membrane in the hollow fiber membrane filter 34, that is, a space between the hollow fiber membrane and a cartridge accommodating the hollow fiber membrane (space through which the filtrate having passed through the hollow fiber membrane flows). Therefore, as illustrated in FIG. 14B, the controller 120 opens only the valve 78, and activates only the pump 62. As a result, the filling liquid in the filling liquid bag 50 flows into the cartridge of the hollow fiber membrane filter 34 through the introduction port 34d, and the filling liquid that has passed through the space in the cartridge flows out from the filtrate discharge port 34c and enters the filtrate tank 44. Thus, the inside of the cartridge of the hollow fiber membrane filter (the outside of the hollow fiber membrane) is washed.

Next, as a third treatment step, the controller 120 executes an air bleeding treatment step of a circuit from the hollow fiber membrane filter 34 to the storage bag 36 (that is, the second connection tube 40). Therefore, as illustrated in FIG. 14C, the controller 120 opens only the valves 70 and 74, and activates only the pump 60. As a result, the filling liquid in the filling liquid bag 54 flows into the hollow fiber membrane filter 34, and the filling liquid flowing out from the hollow fiber membrane filter 34 enters the storage bag 36. Thus, air is removed from the circuit from the hollow fiber membrane filter 34 to the storage bag 36 (the circuit is filled with the filling liquid).

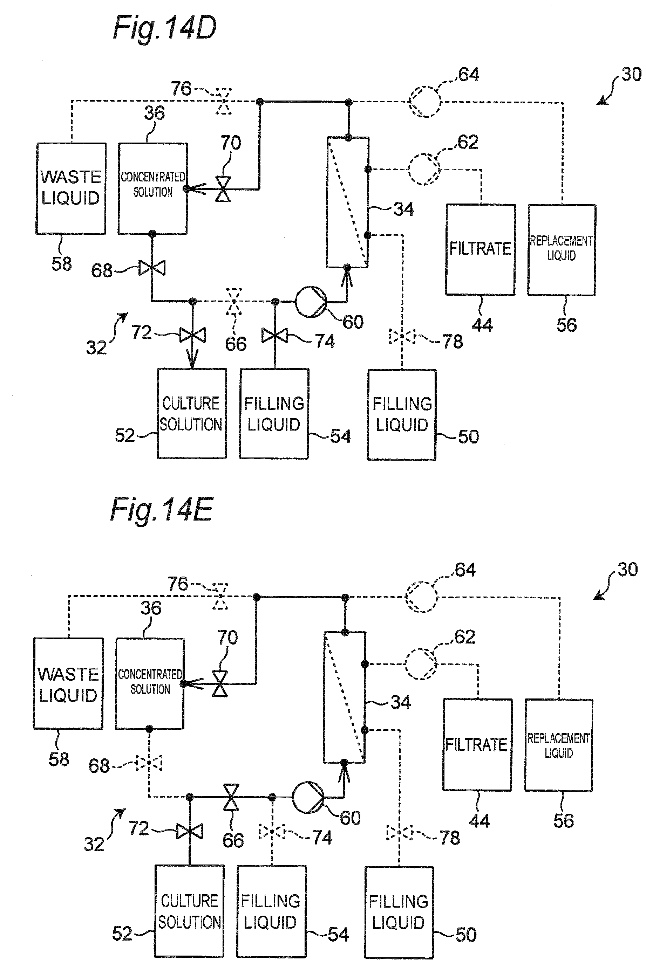

As a fourth treatment step subsequent to the third treatment step, the controller 120 executes an air bleeding treatment step of a circuit from the storage bag 36 to the culture solution tank 52. Therefore, as illustrated in FIG. 14D, the controller 120 opens only the valves 68, 70, 72, and 74, and activates only the pump 60. As a result, the filling liquid in the filling liquid bag 54 passes through the hollow fiber membrane filter 34 and enters the storage bag 36, and the filling liquid in the storage bag 36 enters the culture solution tank 52. As a result, air is removed from the circuit from the storage bag 36 to the culture solution tank 52 (the circuit is filled with the filling liquid).

Next, as a fifth treatment step, the controller 120 executes a treatment step of supplying the cell suspension (culture solution) to be treated by the cell concentration and washing circuit 30 to the storage bag 36. Therefore, as illustrated in FIG. 14E, the controller 120 opens only the valves 66, 70, and 72, and activates only the pump 60. As a result, the cell suspension in the culture solution tank 52 passes through the hollow fiber membrane filter 34 without being filtered, and is stored in the storage bag 36.

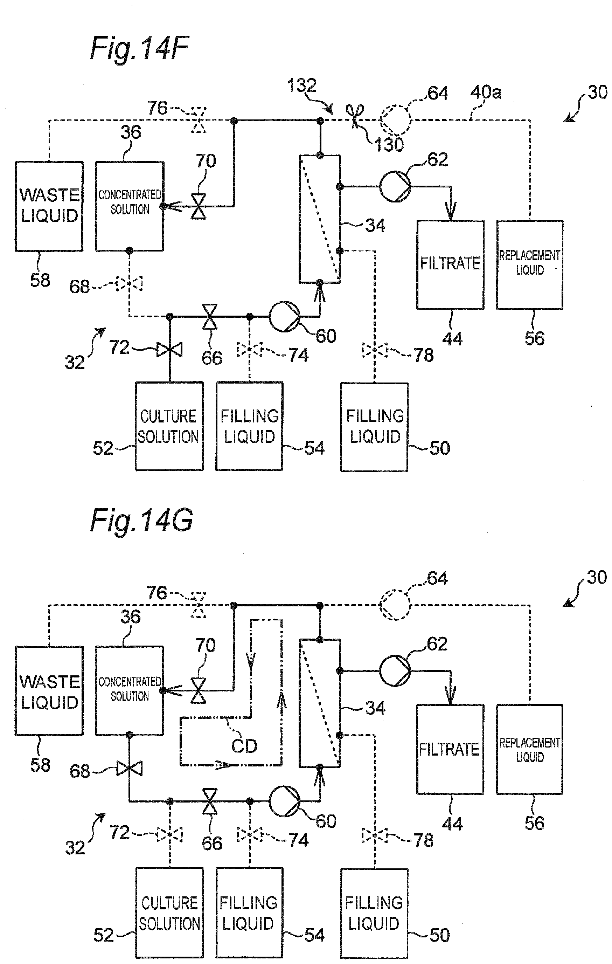

As a sixth treatment step subsequent to the fifth treatment step, the controller 120 subsequently executes a treatment step of supplying the cell suspension (culture solution) to the storage bag 36. At this time, the cell suspension is supplied to the storage bag 36 while being filtered by the hollow fiber membrane filter 34. Therefore, as illustrated in FIG. 14F, the controller 120 opens only the valves 66, 70, and 72, and activates only the pumps 60 and 62. As a result, the cell suspension in the culture solution tank 52 is filtered by the hollow fiber membrane filter 34, and the filtered cell suspension is stored in the storage bag 36.

In the sixth treatment step, the branch tube 40a connected to the second connection tube 40 functions as an accumulator that suppresses pulsation (pressure fluctuation) of the cell suspension from the culture solution tank 52 toward the storage bag 36.

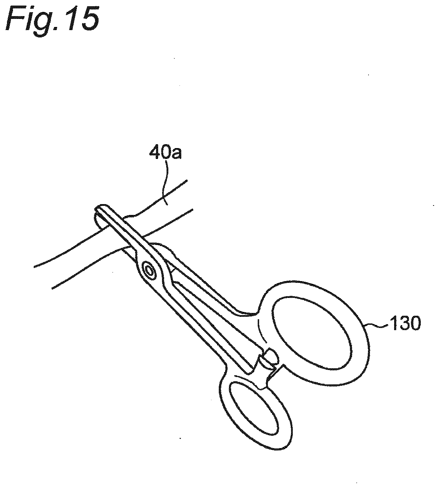

Specifically, when the two pumps 60 and 62 are operated, pulsation occurs in the cell suspension. As a countermeasure, the branch tube 40a connected to the second connection tube 40 is pinched and closed by a pinch device such as forceps 130 as illustrated in FIG. 15 in order to use the branch tube 40a as the accumulator. As a result, a portion 132 from the branch point to the closing position in the branch tube 40a is used as the accumulator. That is, the air in the accumulator 132 absorbs the pressure fluctuation of the cell suspension, and thereby the pressure fluctuation is suppressed.

An effect obtained when the branch tube 40a of the second connection tube 40 functions as the accumulator 132 will be described.

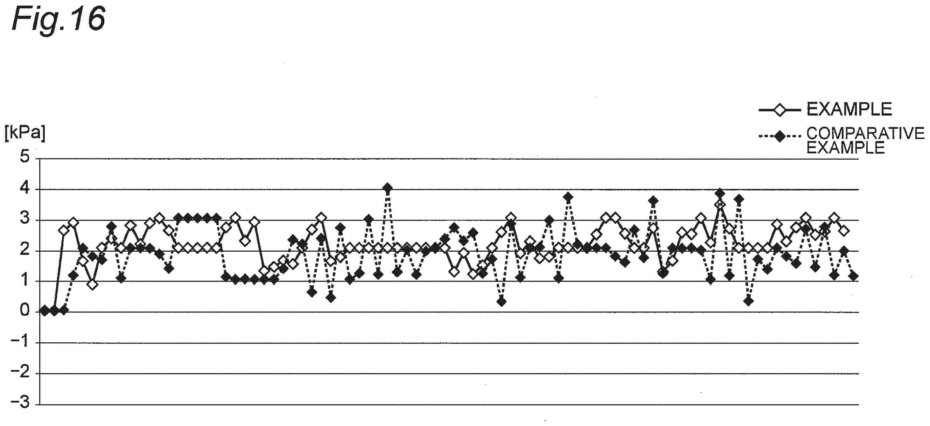

FIG. 16 is a graph illustrating pressure fluctuations of the cell suspension during supply to the circulation circuit in a case where an accumulator is present (example) and in a case where an accumulator is not present (comparative example).

The pressure illustrated in FIG. 16 is the pressure at the inlet port 34a of the hollow fiber membrane filter 34, that is, the pressure detected by the pressure sensor 80. As illustrated in FIG. 16, the pressure fluctuates in both the case where the accumulator is present (example) and the case where the accumulator is not present (comparative example), but the fluctuation range is larger in the comparative example. When the 3σ value (3 sigma value) is calculated, the 3σ value is about 1.84 kPa in the example, and is about 2.65 kPa in the comparative example. That is, the degree of pressure fluctuation of the cell suspension is smaller in the example.

By such an accumulator 132, pulsation (pressure fluctuation) of the cell suspension flowing from the culture solution tank 52 toward the storage bag 36 is suppressed, and thereby the damage of the cells in the cell suspension due to the pulsation is suppressed.

In order to suppress the inflow of the cell suspension into the accumulator 132 (that is, the branch tube 40a), the branch tube 40a extends from the branch point in an obliquely rearward direction with respect to the flow direction of the cell suspension passing through the branch point, as illustrated in a region A of FIG. 10.

In addition, the pinch device that pinches and closes the branch tube 40a in order to cause the branch tube 40a to function as the accumulator 132 is not limited to the forceps 130 illustrated in FIG. 15. The pinch device may be, for example, a pinch valve.

In a case where the pinch device is the forceps 130, it is possible to change the portion of the branch tube 40a to be pinched and closed by the forceps 130. Therefore, even if the pulsation mode of the cell suspension is different, when the forceps 130 pinch and close the appropriate portion of the branch tube 40a, the pulsation can be appropriately suppressed. Preferably, a scale may be added to the branch tube 40a, for example, in order to record the portion of the branch tube 40a pinched by the forceps 130. As a result, the branch tube 40a includes a plurality of indicators indicating a plurality of pinch positions to be pinched by the forceps 130. By recording the portion of the branch tube 40a that is pinched by the forceps 130, substantially identical cell suspension treatment can be performed with high reproducibility.

When a predetermined amount of the cell suspension is stored in the storage bag 36 by the sixth treatment step, that is, when the weight sensor 98 detects a predetermined weight corresponding to the predetermined amount, the controller 120 ends the sixth treatment step, and starts a seventh treatment step. As illustrated in FIG. 14G, in the seventh treatment step, the cell suspension is concentrated by filtration through the hollow fiber membrane filter 34 while being circulated in the circulation circuit 32. Therefore, the controller 120 opens only the valves 66, 68, and 70, and activates only the pumps 60 and 62. As a result, the cell suspension in the storage bag 36 is filtered by the hollow fiber membrane filter 34, and the concentrated cell suspension by the filtration returns to the storage bag 36. During this seventh treatment step, the weight of the storage bag 36 is reduced by the concentration of the cell suspension. The filtrate is sent to the filtrate tank 44.

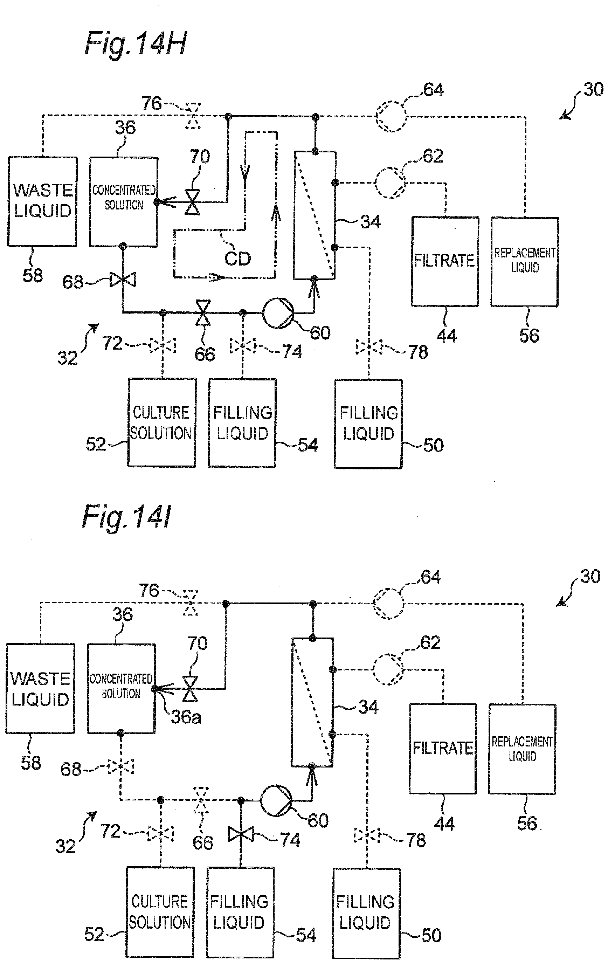

When the cell suspension reaches a predetermined concentration by the seventh treatment step, that is, when the weight sensor 98 detects a predetermined weight corresponding to the predetermined concentration, the controller 120 ends the seventh treatment step, and starts an eighth treatment step. As illustrated in FIG. 14H, in the eighth treatment step, the cell suspension is circulated in the circulation circuit 32 without being filtered by the hollow fiber membrane filter 34. This eighth treatment step is executed for several minutes, for example one minute. Therefore, the controller 120 opens only the valves 66, 68, and 70, and activates only the pump 60.

When the eighth treatment step is completed, the controller 120 stops the pump 60 to end the circulation of the cell suspension in the circulation circuit 32. At this time, the concentrated cell suspension, that is, cells remain in the circulation circuit 32, that is, the first and second connection tubes 38 and 40.

A ninth treatment step is performed in order to collect the cells remaining in the first and second connection tubes 38 and 40 into the storage bag 36. In the ninth treatment step, as illustrated in FIG. 14I, the controller 120 opens only the valves 70 and 74, and activates only the pump 60. As a result, the filling liquid in the filling liquid bag 54 is supplied into the circulation circuit 32, and the supplied filling liquid passes through the hollow fiber membrane filter 34 and flows toward the storage bag 36. The filling liquid flowing in this way pushes the cell suspension, which remains in a portion of the circulation circuit 32 from the pump 60 to the inlet port 36a of the storage bag 36, to flow toward the storage bag 36. As a result, the cells remaining in the circulation circuit 32 after the eighth treatment step, that is, after the concentration treatment, are collected in the storage bag 36.

When the filling liquid that is supplied to the circulation circuit 32 in order to collect the cells remaining in the circulation circuit 32 enters the storage bag 36, the concentration of the cell suspension concentrated to a predetermined concentration in the storage bag 36 is decreased. Therefore, immediately before the filling liquid enters the storage bag 36, the supply of the filling liquid to the circulation circuit 32 is stopped (the pump 60 is stopped and the valve 74 is closed).

For example, a filling liquid arrival time required for the filling liquid in the filling liquid bag 54 to reach the storage bag 36 is calculated in advance based on the discharge capacity of the pump 60, the flow path length from the filling liquid bag 54 to the storage bag 36, the inner diameter of the tube, and the like. When the filling liquid arrival time has elapsed from the supply timing of the filling liquid in the filling liquid bag 54 to the circulation circuit 32, the supply of the filling liquid is stopped.

For example, in a case where the cell suspension and the filling liquid are distinguishable, an optical sensor for detecting the color and/or turbidity of the cell suspension in the circulation circuit 32 (that is, the second connection tube 40) is provided near the inlet port 36a of the storage bag 36.

In a case where the cell suspension is colored in red by phenol red and the filling liquid is transparent, when the filling liquid reaches a portion near the inlet port 36a of the storage bag 36 which is a detection region of the optical sensor, the optical sensor detects a change in color (change from red to a different color) of the cell suspension.

In addition, in a case where the cell suspension that is concentrated to have a predetermined concentration has high turbidity, when the filling liquid reaches a portion near the inlet port 36a of the storage bag 36 which is a detection region of the optical sensor, the optical sensor detects a change in turbidity.

When the optical sensor detects a change in color and/or turbidity of the cell suspension, the controller 120 stops the pump 60 to stop the supply of the filling liquid from the filling liquid bag 54 to the circulation circuit 32.

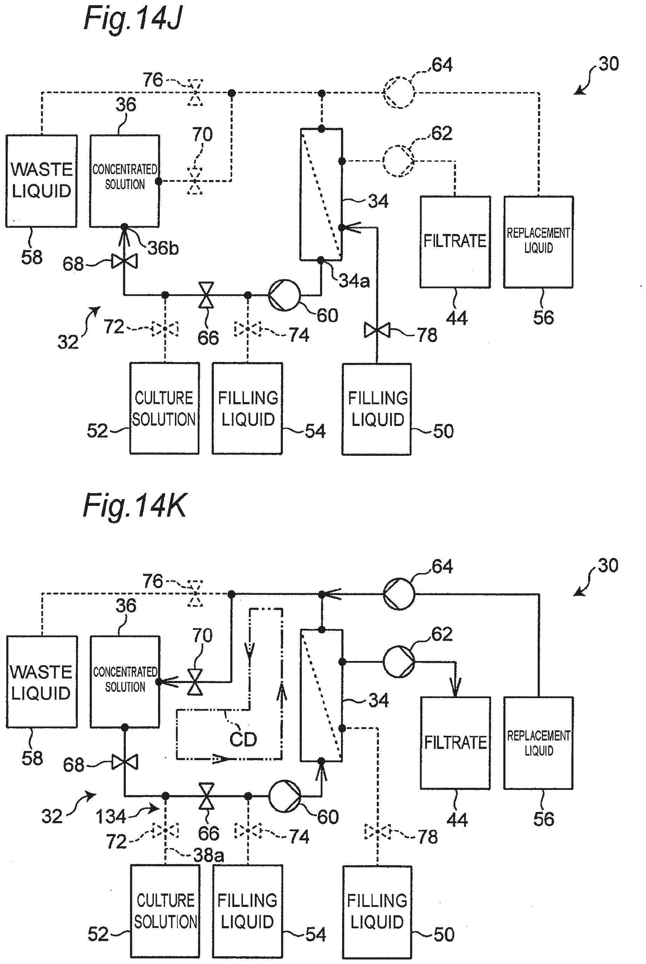

When collecting the cells, which remain in the portion of the circulation circuit 32 from the pump 60 to the inlet port 36a of the storage bag 36, into the storage bag 36 is completed, the controller 120 executes a tenth treatment step. In the tenth treatment step, the cells remaining in the portion of the circulation circuit 32 from the outlet port 36b of the storage bag 36 to the pump 60 are collected. Therefore, as illustrated in FIG. 14J, the controller 120 opens only the valves 66, 68, and 78, and activates only the pump 60. However, the controller 120 reversely drives the pump 60 (the rotor of the roller pump is reversed) in order to push the cell suspension to flow in a direction opposite to the flow direction (circulation direction CD) at the time of the cell concentration step (the seventh treatment step illustrated in FIG. 14G). Thus, the filling liquid in the filling liquid bag 50 is supplied to the hollow fiber membrane filter 34, the supplied filling liquid flows out from the inlet port 34a of the hollow fiber membrane filter 34, and the filling liquid that has flowed out flows toward the outlet port 36b of the storage bag 36. The filling liquid flowing in this way pushes the cell suspension, which remains in the portion of the circulation circuit 32 from the outlet port 36b of the storage bag 36 to the pump 60, to flow toward the storage bag 36. As a result, the cells remaining in the circulation circuit 32 after the eighth treatment step, that is, after the concentration treatment, are collected in the storage bag 36.

By a method (for example, a method using an optical sensor) similar to the ninth treatment step, the supply of the filling liquid in the filling liquid bag 50 to the circulation circuit 32 is stopped before the filling liquid enters the storage bag 36 through the outlet port 36b.

When collecting the cells remaining in the circulation circuit 32 is ended (when the ninth and tenth treatment steps are ended), the controller 120 executes the washing treatment of cells as an eleventh treatment step. Therefore, as illustrated in FIG. 14K, the controller 120 opens only the valves 66, 68, and 70, and activates only the pumps 60, 62, and 64. As a result, the replacement liquid of the replacement liquid bag 56 is supplied to the circulation circuit 32 in which the concentrated cell suspension is circulated, and a mixed solution of the cell suspension and the replacement liquid is filtered by the hollow fiber membrane filter 34. Eventually, the medium components of the cell suspension are replaced with the replacement liquid, and the cells are washed. When the eleventh treatment step is ended, all the steps of the concentration treatment and the washing treatment of cells by the controller 120 are completed.

In the eleventh treatment step, the branch tube 38a of the first connection tube 38 functions as an accumulator that suppresses pulsation (pressure fluctuation) of the cell suspension circulated in the circulation circuit 32 together with the replacement liquid.

Specifically, as illustrated in FIG. 14K, when the three pumps 60, 62, and 64 are operated, pulsation occurs in the cell suspension circulated in the circulation circuit 32. As a countermeasure, the branch tube 38a connected to the first connection tube 38 is pinched and closed by the valve 72 which is a pinch device in order to cause the branch tube 38a to function as the accumulator. As a result, a portion 134 from the branch point to the closing position in the branch tube 38a is used as the accumulator.

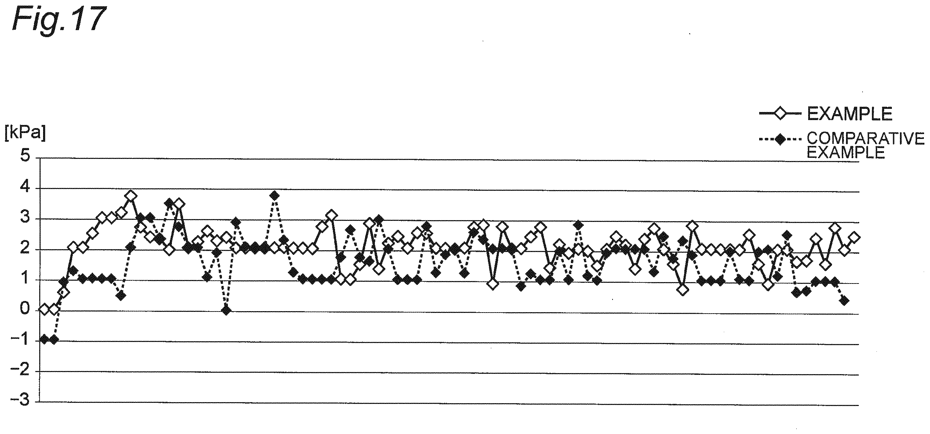

FIG. 17 is a graph illustrating pressure fluctuations of the cell suspension during cell washing in a case where an accumulator is present (example) and in a case where an accumulator is not present (comparative example).

The pressure illustrated in FIG. 17 is the pressure at the inlet port 34a of the hollow fiber membrane filter 34, that is, the pressure detected by the pressure sensor 80. As illustrated in FIG. 17, the pressure fluctuates in both the case where the accumulator is present (example) and the case where the accumulator is not present (comparative example), but the fluctuation range is larger in the comparative example. When the 3σ value (3 sigma value) is calculated, the 3σ value is about 1.91 kPa in the example, and is about 2.54 kPa in the comparative example. That is, the degree of pressure fluctuation of the cell suspension is smaller in the example.

By such an accumulator 134, pulsation (pressure fluctuation) of the cell suspension during the cell washing is suppressed, and thereby the damage of the cells in the cell suspension due to the pulsation is suppressed.

In order to suppress the inflow of the cell suspension into the accumulator 134 (that is, the branch tube 38a), the branch tube 38a extends from the branch point in an orthogonal direction with respect to the flow direction of the cell suspension passing through the branch point, as illustrated in a region B of FIG. 10.

In addition, the pinch device that pinches and closes the branch tube 38a in order to cause the branch tube 38a to function as the accumulator 134 may be forceps.

In a case where the pinch device that pinches the branch tube 38a is the forceps, it is possible to change the portion of the branch tube 38a to be pinched and closed by the forceps. Further, preferably, a scale may be added to the branch tube 38a, for example, in order to record the portion of the branch tube 38a pinched by the forceps. As a result, the branch tube 38a includes a plurality of indicators indicating a plurality of pinch positions to be pinched by the forceps.

According to the present embodiment, it is possible to collect cells remaining in the circulation circuit after the concentration treatment is completed, in the concentration treatment of cells using the circulation circuit including at least the reservoir, the pump, and the hollow fiber membrane filter.

According to the present embodiment, it is possible to appropriately dispose a necessary container with respect to the circulation circuit while making the installation space compact, in the cell suspension treatment using the circulation circuit including at least the reservoir, the pump, and the hollow fiber membrane filter.

Specifically, since the plurality of containers used in the cell suspension treatment are a plurality of bags, and the plurality of bags are suspended and supported, the installation space (footprint) of the cell suspension treatment apparatus 10 can be made compact (compared to a case where the bag is installed in a laid state or a case where the plurality of containers containing the cell suspension and the like are not the bags).

Furthermore, according to the present embodiment, in the concentration treatment and the washing treatment of cells using the circulation circuit including at least the reservoir, the pump, and the hollow fiber membrane filter, the pulsation of the cell suspension flowing in the circulation circuit can be suppressed. As a result, the damage of the cells in the cell suspension can be suppressed.

Specifically, as illustrated in FIG. 14F, when the cell suspension is supplied from the culture tank 52 as the cell suspension supply source to the circulation circuit 32, the branch tube 40a closed by the forceps 130 as the pinch device is used as the accumulator (the accumulator 132 is formed). The accumulator 132 suppresses the pulsation of the cell suspension supplied to the circulation circuit 32.

As illustrated in FIG. 14K, when the replacement liquid is supplied from the replacement liquid bag 56 as the replacement liquid supply source to the circulation circuit 32, the branch tube 38a closed by the valve 72 as the pinch device is used as the accumulator (the accumulator 134 is formed). The accumulator 134 suppresses the pulsation of the cell suspension circulated in the circulation circuit 32 together with the replacement liquid.

Although the present invention has been described with reference to the embodiments, embodiments of the present invention are not limited thereto.

For example, in the case of the embodiment, as illustrated in FIG. 14J, in order to collect the cells remaining in the portion of the circulation circuit 32 from the outlet port 36b of the storage bag 36 to the pump 60, the filling liquid in the filling liquid bag 50 as the filling liquid supply source is supplied to the hollow fiber membrane filter 34. However, the embodiment of the present invention is not limited thereto. The filling liquid supply source may be connected to a portion of the circulation circuit 32, which is between the pump 60 and the valve 70.

In addition, in the case of the embodiment, for example, as illustrated in FIG. 10, the plurality of bags 36, 50, 54, 56, and 58 are suspended from the two arm portions 16A and 16B. However, the embodiment of the present invention is not limited thereto. For example, the number of arm portions from which the plurality of bags are suspended may be one.

Furthermore, as illustrated in FIG. 10, the filling liquid bag 50 and the replacement liquid bag 56 are suspended from the first arm portion 16A, and the waste liquid bag 58, the filling liquid bag 54, and the storage bag 36 are suspended from the second arm portion 16B. However, the arrangement of the plurality of bags is not limited thereto. When the wiring pattern of the connection tubes is changed, the arrangement of the plurality of bags may be changed correspondingly. However, it is preferable to dispose the bag having a large size and a heavy weight, that is, the storage bag 36 and the replacement liquid bag 56 on the outermost side in consideration of ease of hanging.

Furthermore, in the case of the embodiment, as illustrated in the region A of FIG. 10, the branch tube 40a extends from the branch point in an obliquely rearward direction with respect to the flow direction of the cell suspension passing through the branch point with the connection tube 40. As illustrated in the region B of FIG. 10, the branch tube 38a extends from the branch point in an orthogonal direction with respect to the flow direction of the cell suspension passing through the branch point with the connection tube 38. Thus, the inflow of the cell suspension into the branch tubes 38a and 40a functioning as the accumulator is suppressed. However, the embodiment of the present invention is not limited thereto. That is, when the branch tubes 38a and 38b extend from the branch point in an obliquely rearward direction, an orthogonal direction, or an upward direction with respect to the flow direction of the cell suspension flowing through the branch point, the inflow of the cell suspension can be suppressed.

The illustrated embodiment will be roughly described again as follows.

The "main body of the cell suspension treatment apparatus" includes a filtration filter 34 at substantially the center on the front surface. For example, as illustrated in the reference drawing (FIG. 10), various bags are suspended from the apparatus main body, and the various bags and the filtration filter are piped using a tube material to constitute the cell suspension treatment apparatus. The cell suspension treatment apparatus is an apparatus for performing a concentration treatment and a washing treatment on a cell suspension in which cells such as platelets and megakaryocytes are suspended in a liquid medium, and is used, for example, as follows. First, the primer agent stored in the bags 50 and 54 is circulated to wash the inside of the filter and the pipe (the primer agent after use is collected in the bag 58 as a waste liquid). Next, the cultured cell suspension (culture solution) is supplied to the bag 36 from the outside via the tube 38a. Then, the cell suspension is filtered (concentrated) by the filter 34 while being circulated in the circulation circuit 32 (the filtrate is sent to the filtrate tank 44). Finally, the replacement liquid in the bag 56 is supplied while the concentrated cell suspension is circulated in the circulation circuit 32, and the medium component of the cell suspension is replaced with the replacement liquid. In this way, all the steps of the concentration treatment and the washing treatment of cells are completed.

As described above, the plurality of embodiments have been described as examples of the technique in the present invention. Therefore, the accompanying drawings and the detailed description have been provided.

Therefore, the components described in the accompanying drawings and the detailed description may include not only essential components for solving the problem but also non-essential components for solving the problem in order to illustrate the technique. Therefore, it should not be immediately recognized that the non-essential components are essential based on the fact that the non-essential components are described in the accompanying drawings and the detailed description.

In addition, since the embodiments are intended to illustrate the technique in the present invention, various changes, replacements, additions, omissions, and the like can be made within the scope of the claims or equivalents thereof.

The present invention is applicable to a apparatus for treating a cell suspension using a circulation circuit.

## Claims

1. A cell suspension treatment apparatus that performs a concentration treatment on a cell suspension, the cell suspension treatment apparatus comprising:
a circulation circuit in which the cell suspension is circulated; and
a first filling liquid supply source that supplies a filling liquid to the circulation circuit,
wherein the circulation circuit includes
a hollow fiber membrane filter that filters and concentrates the cell suspension,
a reservoir that includes an inlet port and an outlet port, and stores the cell suspension,
a pump that is provided on a downstream side of the reservoir in a circulation direction of the cell suspension and on an upstream side of the hollow fiber membrane filter in the circulation direction, and circulates the cell suspension, and
a first valve that is provided on the downstream side of the reservoir in the circulation direction and on an upstream side of the pump in the circulation direction,
the first filling liquid supply source is connected to a portion of the circulation circuit between the first valve and the pump, and
after the concentrated cell suspension is stored in the reservoir, in a state where the first valve is closed and the pump is driven in a forward direction, the first filling liquid supply source starts supplying the filling liquid to the circulation circuit to push the cell suspension, which remains in a portion of the circulation circuit from the pump to the inlet port of the reservoir, to flow toward the reservoir in the circulation direction by the filling liquid.

2. The cell suspension treatment apparatus according to claim 1, further comprising:
a first optical sensor that is provided near the inlet port of the reservoir, and detects a color and/or turbidity of the cell suspension,
wherein when the first optical sensor detects a change in color and/or turbidity of the cell suspension, the pump is stopped, and the first filling liquid supply source stops supplying the filling liquid to the circulation circuit.

3. The cell suspension treatment apparatus according to claim 1,
wherein the first filling liquid supply source is a filling liquid supply source for priming the circulation circuit and the hollow fiber membrane filter.

4. The cell suspension treatment apparatus according to claim 1, further comprising:
a second filling liquid supply source that supplies a filling liquid to the circulation circuit; and
a second valve that is provided on a downstream side of the hollow fiber membrane filter in the circulation direction and on an upstream side of the reservoir in the circulation direction,
wherein the second filling liquid supply source is connected to a portion of the circulation circuit between the pump and the second valve, and
after the concentrated cell suspension is stored in the reservoir, in a state where the second valve is closed and the pump is driven in a reverse direction, the second filling liquid supply source starts supplying the filling liquid to the circulation circuit to push the cell suspension, which remains in a portion of the circulation circuit from the outlet port of the reservoir to the pump, to flow toward the reservoir in a direction opposite to the circulation direction by the filling liquid.

5. The cell suspension treatment apparatus according to claim 4, further comprising:
a second optical sensor that is provided near the outlet port of the reservoir, and detects a color and/or turbidity of the cell suspension,
wherein when the second optical sensor detects a change in color and/or turbidity of the cell suspension, the pump is stopped, and the second filling liquid supply source stops supplying the filling liquid to the circulation circuit.

6. The cell suspension treatment apparatus according to claim 4,
wherein the second filling liquid supply source is a filling liquid supply source for priming the hollow fiber membrane filter positioned between the pump and the second valve by supplying the filling liquid into the hollow fiber membrane filter.

7. A cell suspension treatment apparatus that treats a cell suspension, the cell suspension treatment apparatus comprising:
a pillar portion;
an arm portion that extends from the pillar portion in a lateral direction;
a cell treatment circuit that is provided on the pillar portion, and includes a circulation circuit that includes at least a storage bag that stores the cell suspension, a pump, and a hollow fiber membrane filter, and concentrates the cell suspension; and
a plurality of hooks which are provided on the arm portion in a state of being arranged side by side in an extending direction of the arm portion, and by which the storage bag and a plurality of bags included in the cell treatment circuit and connected to the circulation circuit are suspended.

8. The cell suspension treatment apparatus according to claim 7,
wherein at least one of the plurality of hooks is movable in the extending direction of the arm portion.

9. The cell suspension treatment apparatus according to claim 7,
wherein at least one of the plurality of hooks is provided on the arm portion via a weight sensor.

10. The cell suspension treatment apparatus according to claim 7, further comprising:
a drain pan that is disposed immediately below the bag suspended by each of the plurality of hooks.

11. The cell suspension treatment apparatus according to claim 7,
wherein the cell treatment circuit is provided on a front surface of the pillar portion.

12. The cell suspension treatment apparatus according to claim 7, further comprising:
a plurality of casters; and
a handle for a user to operate when the user moves the cell suspension treatment apparatus via the plurality of casters,
wherein the handle functions as a guard that extends to a side of a tip end of the arm portion to avoid contact between the user and the bag on an outermost side.

13. The cell suspension treatment apparatus according to claim 7,
wherein the plurality of bags include a filling liquid bag that supplies a filling liquid for priming the circulation circuit to the circulation circuit, a waste liquid bag that collects the filling liquid after priming, and a replacement liquid bag that supplies a replacement liquid for washing cells of the concentrated cell suspension stored in the storage bag, to the circulation circuit.

14. A cell suspension treatment apparatus that perform a concentration treatment and a washing treatment of cells, the cell suspension treatment apparatus comprising:
a circulation circuit that includes
a reservoir that stores a cell suspension,
a hollow fiber membrane filter that filters and concentrates the cell suspension,
a first connection tube that connects an outlet port of the reservoir to an inlet port of the hollow fiber membrane filter, and
a second connection tube that connects an outlet port of the hollow fiber membrane filter to an inlet port of the reservoir;
a first branch tube that branches from the first connection tube and is connected to a cell suspension supply source;
a second branch tube that branches from the second connection tube and is connected to a replacement liquid supply source;
a roller pump that is provided in the first or second connection tube;
a first pinch device that pinches the first branch tube; and
a second pinch device that pinches the second branch tube,
wherein when the cell suspension is supplied from the cell suspension supply source to the circulation circuit, the second pinch device pinches and closes the second branch tube, and
when the replacement liquid is supplied from the replacement liquid supply source to the circulation circuit, the first pinch device pinches and closes the first branch tube.

15. The cell suspension treatment apparatus according to claim 14,
wherein at least one of the first and second branch tubes extends from a branch point of the first or second branch tube in an orthogonal direction with respect to a flow direction of the cell suspension passing through the branch point.

16. The cell suspension treatment apparatus according to claim 14,
wherein at least one of the first and second branch tubes extends from a branch point of the first or second branch tube in an obliquely rearward direction with respect to a flow direction of the cell suspension passing through the branch point.

17. The cell suspension treatment apparatus according to claim 14,
wherein at least one of the first and second branch tubes extends from a branch point of the first or second branch tube in an upward direction with respect to a flow direction of the cell suspension passing through the branch point.

18. The cell suspension treatment apparatus according to claim 14,
wherein each of the first and second pinch devices is a pinch valve or forceps.

19. The cell suspension treatment apparatus according to claim 14,
wherein the first and second branch tubes include a plurality of indicators indicating a plurality of pinch positions.
